# EUROPEAN PATENT APPLICATION

(11) **EP 4 649 942 A1**
(43) Date of publication of application: **19.11.2025**
(21) Application number: 24741257.0
(22) Date of filing: 09.01.2024
(51) Int. Cl.: A61K 9/107, A61K 9/20, A61K 9/48, A61K 31/416, A61P 35/00, A61P 27/02, A61P 19/02, A61P 17/06

(54) **SELF-EMULSIFYING PHARMACEUTICAL COMPOSITION, PREPARATION METHOD THEREFOR, AND APPLICATION THEREOF**

(30) Priority: 09.01.2023 CN 202310025473; 22.09.2023 CN 202311229103
(71) Applicant: Shanghai Runshi Medical Technology Co., Ltd, Shanghai 201318 (CN); Shanghai Institute of Materia Medica, Chinese Academy of Sciences, Shanghai 201203 (CN)
(72) Inventor: LI, Yaping, Shanghai 201203 (CN); HE, Yuxia, Shanghai 201318 (CN); SHENG, Lidan, Shanghai 201318 (CN); DING, Jian, Shanghai 201203 (CN); DUAN, Wenhu, Shanghai 201203 (CN); CHEN, Xuetao, Shanghai 201318 (CN); GENG, Jiangtao, Shanghai 201318 (CN); FENG, Xue, Shanghai 201318 (CN); WANG, Xiaoyan, Shanghai 201318 (CN); XIE, Hua, Shanghai 201203 (CN); CHEN, Lingli, Shanghai 201203 (CN); GU, Wangwen, Shanghai 201203 (CN)
(74) Representative: Barrow, Nicholas Martin
(86) International application number: PCT/CN2024/071415
(87) International publication number: WO 2024/149260

(57) **Abstract**

A self-emulsifying pharmaceutical composition of compound (I), which improves the solubility and oral bioavailability of the medicine, has stable properties, and can be made into a plurality of dosage forms such as oral liquids, capsules, and tablets.

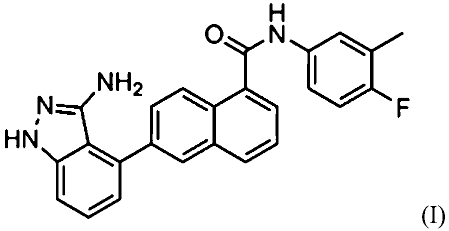

## Description

### Technical Field

The present invention belongs to the field of medicine, and specifically relates to a self-emulsifying pharmaceutical composition and preparation process and use thereof.

### Background of Art

Compound (I) was first disclosed in CN104860885A, and its structural formula is shown in the following formula (I):

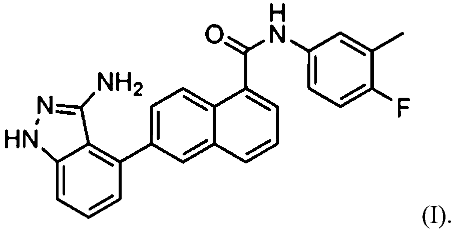

Compound (I) is a VEGFR/CSF1R dual inhibitor with excellent activity, which can inhibit tumour cell angiogenesis and promote tumour immunity to exert anti-tumour effects. Compound (I) can be used to prepare drugs for preventing and/or treating abnormal vascular proliferation-related diseases, and/or drugs as VEGFR-2 inhibitors, and/or drugs for treating CSF1R kinase signal transduction pathway-related diseases, and/or drugs as CSF1R kinase inhibitors. The abnormal vascular proliferation-related disease is selected from tumour, rheumatoid arthritis, age-related macular degeneration, and psoriasis; said tumour includes lung cancer, breast cancer, colorectal cancer, prostate cancer, pancreatic cancer, stomach cancer, liver cancer, ovarian cancer, renal cancer, glioma (e.g., glioblastoma, astroglioma, medulloblastoma), melanoma, pancreatic cancer, head and neck cancer, bladder cancer, cervix cancer, cholangiocarcinoma, nasopharyngeal carcinoma, thyroid carcinoma, osteosarcoma, synovial sarcoma, rhabdomyosarcoma, fibrosarcoma, leiomyosarcoma, myeloma, brain glioma, brain metastasis (metastatic brain tumor), meningioma, and lymphadenoma.

### Summary of the Invention

### Technical problems

Compound (I) has very poor solubility in water, which results in a very low drug oral availability when it is developed into common dosage forms such as oral liquids, tablets, capsules, and other common oral preparations.

Emulsion is a dosage form commonly used to improve the absorption of lipid soluble drugs. However, conventional emulsions are not well accepted due to their large volume, short shelf life, and poor palatability.

The technical problem to be solved by the present invention is to provide a self-emulsifying pharmaceutical composition of Compound (I), wherein the preparation prepared by the self-emulsifying pharmaceutical composition can have higher drug solubility and oral bioavailability, and the self-emulsifying pharmaceutical composition is stable in properties and can be prepared into various dosage forms such as oral liquids, capsules, and tablets, which are convenient for patients to take.

### Technical solutions

In order to solve the above technical problem, the present invention provides the following technical solutions:
On the one hand, the present invention provides a self-emulsifying pharmaceutical composition of Compound (I), comprising: Compound (I), an oil phase, an emulsifier and a co-emulsifier, wherein the weight ratio of Compound (I):the oil phase:the emulsifier:the co-emulsifier is 0.5-2:0-20:50-80:20-35,
Compound (I) has a structural formula represented by the following formula (I),

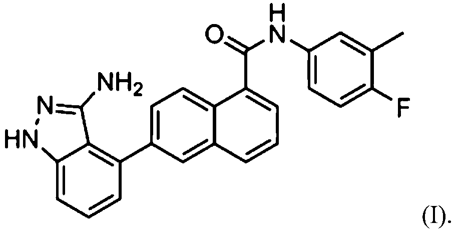

In some embodiments of the present invention, the weight ratio of Compound (I):the oil phase:the emulsifier:the co-emulsifier is 0.99-1.98:0-20:50-80:20-35.

In some embodiments of the present invention, the weight ratio of Compound (I):the oil phase:the emulsifier:the co-emulsifier is 0.99:0-20:50-80:20-35.

In some embodiments of the present invention, the weight ratio of Compound (I):the oil phase:the emulsifier:the co-emulsifier is 1.25:0-20:50-80:20-35.

In some embodiments of the present invention, the weight ratio of Compound (I):the oil phase:the emulsifier:the co-emulsifier is 1.98:0-20:50-80:20-35.

In some embodiments of the present invention, the weight ratio of Compound (I):the oil phase:the emulsifier:the co-emulsifier is 0.5-2:0-15:55-75:20-30, preferably 0.99-1.98:0-15:55-75:20-30, more preferably 0.99-1.98:0-15:55-75:24-30.

In some embodiments of the present invention, the weight ratio of Compound (I):the oil phase:the emulsifier:the co-emulsifier is 0.5-2:10-20:50-65:20-30, preferably 0.99-1.98:10-15:55-65:25-30, more preferably 0.99-1.98:13-14:55-57:28-29.

In some embodiments of the present invention, the weight ratio of Compound (I):the oil phase:the emulsifier:the co-emulsifier is 0.99:10-20:50-65:20-30, preferably 0.99:10-15:55-65:25-30, further preferably 0.99:13-14:55-57:28-29, still further preferably 0.99:13.86:56.44:28.71.

In some embodiments of the present invention, the weight ratio of Compound (I):the oil phase:the emulsifier:the co-emulsifier is 1.25:10-20:50-65:20-30, preferably 1.25:10-15:55-65:25-30, further preferably 1.25:13-14:55-57:28-29, still further preferably 1.25:13.83:56.25:28.67.

In some embodiments of the present invention, the weight ratio of Compound (I):the oil phase:the emulsifier:the co-emulsifier is 1.98:10-20:50-65:20-30, preferably 1.98:10-15:55-65:25-30, further preferably 1.98:13-14:55-57:28-29, still further preferably 1.98:13.72:55.88:28.42.

In some embodiments of the present invention, the weight ratio of Compound (I):the oil phase:the emulsifier:the co-emulsifier is 0.5-2:0:60-80:20-30, preferably 0.99-1.98:0:70-80:20-30, or 0.99-1.98:0:75-80:25-30, or 0.99-1.98:0:70-75:20-25, or 0.99-1.98:0:74-76:24-26.

In some embodiments of the present invention, the weight ratio of Compound (I):the oil phase:the emulsifier:the co-emulsifier is 0.99:0:60-80:20-30, preferably 0.99:0:70-80:20-30, or 0.99:0:75-80:25-30, or 0.99:0:70-75:20-25, or 0.99:0:74-76:24-26, further preferably 0.99:0:74.26:24.75.

In some embodiments of the present invention, the concentration of Compound (I) is 5-21mg/mL, preferably 5-20mg/mL, more preferably 10-21mg/mL, further preferably 10-20mg/mL, still further preferably 10mg/mL, 10.5mg/mL, 20mg/mL, 20.99mg/mL, 13.25mg/mL or 12.5mg/mL.

In some embodiments of the present invention, the concentration of Compound (I) is 5-20mg/g, preferably 9.9-19.8mg/g, more preferably 9.9mg/g, 19.8mg/g or 12.5mg/g.

The oil phase is one of the important excipients in the self-emulsifying preparation. The requirements for the oil phase include safety, stability, the ability to dissolve the formula amount of the drug with a relatively small quantity, and being easily emulsified by the emulsifier in the formula. The use of an oil phase with high drug solubility can increase the drug loading capacity of the self-emulsifying system; a carrier for oil can increase the proportion of drug transport through the lymphatic system, thereby improving the bioavailability of the drug.

In some embodiments of the present invention, the oil phase of the present invention is selected from medium-chain triglycerides (MCT), monocaprylin, dicaprylin, glyceryl monooleate, glyceryl monolinoleate, corn oil, castor oil, sesame oil, peanut oil, olive oil, and the like; preferably medium-chain triglycerides.

A nonionic surfactant with a high HLB value is generally used as an emulsifier in a self-emulsifying preparation. Nonionic surfactants are less toxic than ionic surfactants, and they only cause a reversible change in the permeability of the gastrointestinal tract wall. The strong hydrophilicity of surfactants with a high HLB value is requisite for the immediate formation of oil-in-water emulsion droplets and the diffusion of self-emulsifying liquid in an aqueous solution environment, which can make the self-emulsification process faster. Emulsifiers are amphiphilic and themselves can dissolve relatively large amounts of hydrophobic drugs, which can prevent drug deposition in the gastrointestinal tract and prolong the dissolved state of drug molecules, which is very important for effective absorption. When the surfactant content is as high as a certain level, it will lead to the formation of a self-emulsifying system.

In some embodiments of the present invention, the emulsifier is selected from polyoxyl-40 hydrogenated castor oil (e.g., RH 40, such as Cremophor^{®} RH 40 or Kolliphor^{®} RH 40), polyoxyl-35 castor oil (e.g., Kolliphor EL or Cremophor EL), tocopherol polyethylene glycol succinate (e.g., TPGS 1000), polyoxyl-15 hydroxystearate (e.g., HS 15, such as Kolliphor^{®} HS 15), linoleoyl polyoxyl-6 glyceride, caprylocaproyl polyoxyl glyceride (e.g., Labrasol^{®} ALF) and the like; preferably polyoxyl-40 hydrogenated castor oil, tocopherol polyethylene glycol succinate, and polyoxyl-35 castor oil; more preferably polyoxyl-40 hydrogenated castor oil.

The co-emulsifier in the self-emulsifying preparation helps the active ingredient to form a uniform emulsion and maintain the stability of the emulsion during storage.

In some embodiments of the present invention, the co-emulsifier is selected from polyethylene glycol (PEG), diethylene glycol monoethyl ether (e.g., Transcutol HP), propylene glycol, glycerol; preferably polyethylene glycol 400 (PEG 400), diethylene glycol monoethyl ether (e.g., Transcutol HP).

Further optionally, the self-emulsifying pharmaceutical composition of Compound (I) of the present invention further comprises an antioxidant, preferably, the weight ratio of Compound (I) to the antioxidant is 1:(0-0.5), preferably 1:(0-0.1), more preferably 1:(0-0.05), further preferably 1:(0-0.03), still further preferably 0.99:0.02, 1:0.02 or 1:0. When the self-emulsifying pharmaceutical composition of Compound (I) of the present invention contains an antioxidant, the lower limit of the amount of the antioxidant is not particularly limited, for example, the weight ratio of Compound (I) to the antioxidant may be 1:0.0001, for example 1:0.001. In an embodiment, the self-emulsifying pharmaceutical composition of Compound (I) of the present invention further optionally contains an antioxidant. Preferably, the weight ratio of Compound (I) to the antioxidant is 1:(0 or 0.001-0.5), preferably 1:(0 or 0.001-0.1), more preferably 1:(0 or 0.001-0.05), further preferably 1:(0 or 0.001-0.03), still further preferably 0.99:0.02, 1:0.02 or 1:0.

Herein, the total weight of the self-emulsifying pharmaceutical composition is 100 parts; the weight ratio of components in the pharmaceutical composition is based on the weight parts; the weight parts are allowed to have a certain error range, e.g., ±5, or ±3, or ±2, or ±1, or ±0.5, or ±0.3, or ±0.2, or ±0.1, or ±0.05, or ±0.02, or ±0.01.

In some embodiments of the present invention, the total weight of the self-emulsifying pharmaceutical composition is 100.

When the self-emulsifying pharmaceutical composition of Compound (I) of the present invention contains an antioxidant, the proportions of other excipients such as oil phase, emulsifier and co-emulsifier can be reduced accordingly. For example, based on 1 part by weight of Compound (I), the amount of the antioxidant is determined according to the weight ratio of Compound (I) to the antioxidant, and the amounts of the oil phase, the emulsifier and the co-emulsifier are determined according to the weight ratios of Compound (I) to the oil phase, to the emulsifier and to the co-emulsifier, and then the amount of Compound (I), the amount of the antioxidant, and the amounts of the oil phase, the emulsifier and the co-emulsifier are normalized to obtain the amount of each component based on the total weight of 100; or alternatively based on 1 part by weight of Compound (I), the amount of the antioxidant is determined according to the weight ratio of Compound (I) to the antioxidant, and then the amount of the antioxidant is deducted from the amount of the emulsifier (or the oil phase; or the co-emulsifier), while the amounts of Compound (I), the oil phase, and the co-emulsifier remain unchanged (or the amounts of Compound (I), the emulsifier and the co-emulsifier remain unchanged; or the amount of Compound (I), the oil phase, and the emulsifier remain unchanged).

In some embodiments of the present invention, the antioxidant is selected from butylated hydroxytoluene, butylated hydroxyanisole, vitamin E, and propyl gallate; preferably butylated hydroxytoluene, and vitamin E, more preferably butylated hydroxytoluene.

The above embodiments of the present invention can be combined arbitrarily, and the embodiments obtained by the combination also belong to the embodiments of the present invention.

In some embodiments of the present invention, the self-emulsifying pharmaceutical composition of Compound (I) can be prepared into a self-emulsifying preparation of Compound (I), the dosage form of the self-emulsifying preparation is an oral liquid, a capsule or a tablet, and the capsule can be a soft capsule or a hard capsule.

On the other hand, the present invention also provides a process for preparing the above-mentioned self-emulsifying preparation of Compound (I), which comprises: adding excipients of the self-emulsifying pharmaceutical composition into a liquid preparation tank, heating and stirring evenly, adding Compound (I) and continuing heating and stirring until it is completely dissolved to obtain a self-emulsifying pharmaceutical composition of Compound (I), wherein the excipients of the self-emulsifying pharmaceutical composition include an oil phase, an emulsifier, a co-emulsifier, and optionally, an antioxidant.

The obtained self-emulsifying pharmaceutical composition is packaged into oral liquid bottles or filled into capsules, or the self-emulsifying pharmaceutical composition is prepared into granules or pellets by liquid solidification technology and compressed into tablets or capsules, to obtain a self-emulsifying preparation of Compound (I).

The temperature for heating and stirring is 40-70°C.

The liquid solidification technology can be carried out by conventional methods in the art, for example, a non-volatile carrier such as microcrystalline cellulose, lactose, starch, and hypromellose is mixed and dispersed with a self-emulsifying liquid, and an adsorbent nano-silica is optionally added to absorb the remaining liquid to form granules or micropellets, which can finally be compressed into tablets after the addition of an excipient for tableting such as croscarmellose sodium and magnesium stearate, or can finally be prepared into capsules.

In another aspect, the present invention also provides use of the above self-emulsifying preparation of Compound (I) in manufacture of a drug for preventing and/or treating abnormal vascular proliferation-related diseases, and/or a drug as VEGFR-2 inhibitor, and/or a drug for treating CSF1R kinase signal transduction pathway-related diseases, and/or a drug as CSF1R kinase inhibitor.

In some embodiments of the present invention, the abnormal vascular proliferation-related disease is selected from tumour, rheumatoid arthritis, age-related macular degeneration, and psoriasis; said tumour is preferably selected from lung cancer, breast cancer, colorectal cancer, prostate cancer, pancreatic cancer, stomach cancer, liver cancer, ovarian cancer, renal cancer, glioma, melanoma, pancreatic cancer, head and neck cancer, bladder cancer, cervix cancer, cholangiocarcinoma, nasopharyngeal carcinoma, thyroid carcinoma, osteosarcoma, synovial sarcoma, rhabdomyosarcoma, fibrosarcoma, leiomyosarcoma, myeloma, brain glioma, brain metastasis, meningioma and lymphadenoma. The glioma is preferably glioblastoma, astroglioma, or medulloblastoma.

The self-emulsifying preparation of the present invention can be a solid or liquid dosage form. In case of being diluted with water or in case of being diluted by gastrointestinal fluid during oral administration, the preparation can be emulsified to form an oil-in-water emulsion or microemulsion, which usually has an average particle size of greater than 0 to 250nm, preferably greater than 0 to 100nm, or 10-100 nm, more preferably greater than 0 to 50nm, further preferably 10-50nm, still further preferably 10-40nm, or 20-40nm. When in contact with gastric fluid or intestinal fluid, the self-emulsifying preparation of the present invention can spontaneously form a microemulsion, while Compound (I) is dissolved in the microemulsion. Therefore, the self-emulsifying preparation of the present invention can increase the solubility of Compound (I) as drug in the gastrointestinal tract. Compound (I) as drug exists in these tiny microemulsion droplets and is rapidly distributed throughout the gastrointestinal tract. Compound (I) as drug is distributed between the oil and aqueous phases, and the transmembrane absorption of Compound (I) as drug is greatly improved with the huge specific surface area of tiny oil droplets, thereby improving the oral bioavailability of Compound (I) as drug.

The present invention provides the following technical solutions:
Solution 1. A self-emulsifying pharmaceutical composition of Compound (I), comprising: Compound (I), an oil phase, an emulsifier and a co-emulsifier, wherein the weight ratio of Compound (I):the oil phase:the emulsifier:the co-emulsifier is 0.5-2:0-20:50-80:20-35; preferably, 0.99-1.98:0-20:50-80:20-35; more preferably, 0.99:0-20:50-80:20-35, or 1.25:0-20:50-80:20-35, or 1.98:0-20:50-80:20-35; Compound (I) has a structural formula represented by the following formula (I), 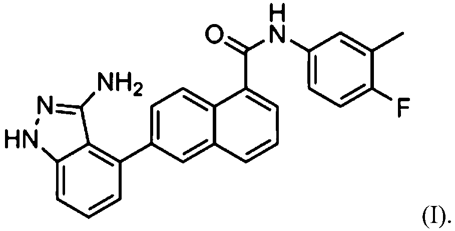
Solution 2. The self-emulsifying pharmaceutical composition of Compound (I) according to solution 1, characterised in that the weight ratio of Compound (I):the oil phase:the emulsifier:the co-emulsifier is
   0.5-2:10-20:50-65:20-30; or
   0.5-2:0:60-80:20-30; or
   0.5-2:0-15:55-75:20-30;
      more preferably
   0.99-1.98:0-15:55-75:20-30; or
   0.99-1.98:0-15:55-75:24-30; or
   0.99-1.98:10-15:55-65:25-30; or
   0.99-1.98:13-14:55-57:28-29; or
   0.99-1.98:0:70-80:20-30; or
   0.99-1.98:0:75-80:25-30; or
   0.99-1.98:0:70-75:20-25; or
   0.99-1.98:0:74-76:24-26;
   further preferably
   0.99:10-20:50-65:20-30; or
   0.99:10-15:55-65:25-30; or
   0.99:13-14:55-57:28-29; or
   0.99:13.86:56.44:28.71; or
   1.25:10-20:50-65:20-30; or
   1.25:10-15:55-65:25-30; or
   1.25:13-14:55-57:28-29; or
   1.25:13.83:56.25:28.67; or
   1.98:10-20:50-65:20-30; or
   1.98:10-15:55-65:25-30; or
   1.98:13-14:55-57:28-29; or
   1.98:13.72:55.88:28.42; or
   0.99:0:60-80:20-30; or
   0.99:0:70-80:20-30; or
   0.99:0:75-80:25-30; or
   0.99:0:70-75:20-25; or
   0.99:0:74-76:24-26; or
   0.99:0:74.26:24.75.
Solution 3. The self-emulsifying pharmaceutical composition of Compound (I) according to any one of the preceding solutions, characterised in that the concentration of Compound (I) is 5-20mg/g, preferably 9.9-19.8mg/g, more preferably 9.9mg/g, 19.8mg/g or 12.5mg/g; or
   the concentration of Compound (I) is 5-21mg/mL, preferably 5-20mg/mL, or 10-21mg/mL, or 10-20mg/mL, further preferably 10mg/mL, 10.5mg/mL, 20mg/mL, 20.99mg/mL, 13.25mg/mL or 12.5mg/mL.
Solution 4. The self-emulsifying pharmaceutical composition of Compound (I) according to any one of the preceding solutions, characterised in that the oil phase is selected from medium-chain triglycerides, monocaprylin, dicaprylin, glyceryl monooleate, glyceryl monolinoleate, corn oil, castor oil, sesame oil, peanut oil, olive oil; preferably medium-chain triglycerides.
Solution 5. The self-emulsifying pharmaceutical composition of Compound (I) according to any one of the preceding solutions, characterised in that the emulsifier is selected from polyoxyl-40 hydrogenated castor oil, polyoxyl-35 castor oil, tocopherol polyethylene glycol succinate, polyoxyl-15 hydroxystearate, linoleoyl polyoxyl-6 glyceride, caprylocaproyl polyoxyl glyceride; preferably polyoxyl-40 hydrogenated castor oil, tocopherol polyethylene glycol succinate, polyoxyl-35 castor oil; more preferably polyoxyl-40 hydrogenated castor oil.
Solution 6. The self-emulsifying pharmaceutical composition of Compound (I) according to any one of the preceding solutions, characterised in that the co-emulsifier is selected from polyethylene glycol, diethylene glycol monoethyl ether, propylene glycol, glycerol; preferably polyethylene glycol 400, diethylene glycol monoethyl ether.
Solution 7. The self-emulsifying pharmaceutical composition of Compound (I) according to any one of the preceding solutions, characterised in that it further optionally contains an antioxidant, preferably, the weight ratio of Compound (I) to the antioxidant is 1:(0-0.5), preferably 1:(0-0.1), more preferably 1:(0-0.05), further preferably 1:(0-0.03), still further preferably 0.99:0.02, 1:0.02 or 1:0;
   more preferably, the weight ratio of Compound (I) to the antioxidant is 1:(0 or 0.001-0.5), preferably 1:(0 or 0.001-0.1), more preferably 1:(0 or 0.001-0.05), further preferably 1:(0 or 0.001-0.03), still further preferably 0.99:0.02, 1:0.02 or 1:0.
Solution 8. The self-emulsifying pharmaceutical composition of Compound (I) according to solution 7, characterised in that the antioxidant is selected from butylated hydroxytoluene, butylated hydroxyanisole, vitamin E, propyl gallate; preferably butylated hydroxytoluene, vitamin E, more preferably butylated hydroxytoluene.
Solution 9. The self-emulsifying pharmaceutical composition of Compound (I) according to solution 1, characterised in that the self-emulsifying pharmaceutical composition is consisted of Compound (I), the oil phase, the emulsifier and the co-emulsifier, wherein the weight ratio of Compound (I):the oil phase:the emulsifier:the co-emulsifier is 0.5-2:10-20:50-65:20-30, preferably, 0.99-1.98:10-15:55-65:25-30, more preferably, 0.99-1.98:13-14:55-57:28-29, e.g., 0.99:13-14:55-57:28-29, or 1.25:13-14:55-57:28-29, or 1.98:13-14:55-57:28-29; further preferably, 0.99:13.86:56.44:28.71, or 1.25:13.83:56.25:28.67, or 1.98:13.72:55.88:28.42; wherein the oil phase is medium-chain triglycerides; the emulsifier is polyoxyl-40 hydrogenated castor oil, tocopherol polyethylene glycol succinate, polyoxyl-15 hydroxystearate or polyoxyl-35 castor oil; the co-emulsifier is polyethylene glycol 400 or diethylene glycol monoethyl ether; or
   the self-emulsifying pharmaceutical composition is consisted of Compound (I), the oil phase, the emulsifier and the co-emulsifier, wherein the weight ratio of Compound (I):the oil phase:the emulsifier:the co-emulsifier is 0.99-1.98:13-14:55-57:28-29, e.g., 0.99:13-14:55-57:28-29; preferably, 0.99:13.86:56.44:28.71; wherein the oil phase is medium-chain triglycerides; the emulsifier is polyoxyl-40 hydrogenated castor oil; the co-emulsifier is polyethylene glycol 400 or diethylene glycol monoethyl ether; or
   the self-emulsifying pharmaceutical composition is consisted of Compound (I), the oil phase, the emulsifier and the co-emulsifier, wherein the weight ratio of Compound (I):the oil phase:the emulsifier:the co-emulsifier is 0.99-1.98:13-14:55-57:28-29, e.g., 0.99:13-14:55-57:28-29; preferably, 0.99:13.86:56.44:28.71; wherein the oil phase is medium-chain triglycerides; the emulsifier is tocopherol polyethylene glycol succinate; the co-emulsifier is polyethylene glycol 400 or diethylene glycol monoethyl ether; or
   the self-emulsifying pharmaceutical composition is consisted of Compound (I), the oil phase, the emulsifier and the co-emulsifier, wherein the weight ratio of Compound (I):the oil phase:the emulsifier:the co-emulsifier is 0.99-1.98:13-14:55-57:28-29, e.g., 0.99:13-14:55-57:28-29; preferably, 0.99:13.86:56.44:28.71; wherein the oil phase is medium-chain triglycerides; the emulsifier is polyoxyl-15 hydroxystearate or polyoxyl-35 castor oil; the co-emulsifier is polyethylene glycol 400; or
   the self-emulsifying pharmaceutical composition is consisted of Compound (I), the oil phase, the emulsifier and the co-emulsifier, wherein the weight ratio of Compound (I):the oil phase:the emulsifier:the co-emulsifier is 0.99-1.98:13-14:55-57:28-29, e.g., 1.25:13-14:55-57:28-29, preferably 1.25:13.83:56.25:28.67; wherein the oil phase is medium-chain triglycerides; the emulsifier is polyoxyl-40 hydrogenated castor oil; the co-emulsifier is polyethylene glycol 400; or
   the self-emulsifying pharmaceutical composition is consisted of Compound (I), the oil phase, the emulsifier and the co-emulsifier, wherein the weight ratio of Compound (I):the oil phase:the emulsifier:the co-emulsifier is 0.99-1.98:13-14:55-57:28-29, e.g., 0.99:13-14:55-57:28-29, or 1.98:13-14:55-57:28-29, preferably, 0.99:13.86:56.44:28.71; or 1.98:13.72:55.88:28.42; wherein the oil phase is medium-chain triglycerides; the emulsifier is polyoxyl-40 hydrogenated castor oil; the co-emulsifier is polyethylene glycol 400; or
   the self-emulsifying pharmaceutical composition is consisted of Compound (I), the oil phase, the emulsifier, the co-emulsifier and the antioxidant, wherein the weight ratio of Compound (I):the oil phase:the emulsifier:the co-emulsifier:the antioxidant is 0.5-2:10-20:50-65:20-30:(greater than 0 to 0.5), preferably, 0.99-1.98:10-15:55-65:25-30:(greater than 0 to 0.5), more preferably, 0.99-1.98:10-15:55-61:27-29:(greater than 0 to 0.5), e.g., 0.99:13-14:55-57:28-29:(0.01-0.03), or 0.99:10-12:59-61:26-28:(0.4-0.5), further preferably 0.99:13.86:56.42:28.71:0.02, or 0.99:10.90:60.39:27.22:0.50; wherein the oil phase is medium-chain triglycerides; the emulsifier is polyoxyl-40 hydrogenated castor oil, tocopherol polyethylene glycol succinate, polyoxyl-15 hydroxystearate or polyoxyl-35 castor oil; the co-emulsifier is polyethylene glycol 400 or diethylene glycol monoethyl ether; the antioxidant is butylated hydroxytoluene, or vitamin E; or
   the self-emulsifying pharmaceutical composition is consisted of Compound (I), the oil phase, the emulsifier, the co-emulsifier and the antioxidant, wherein the weight ratio of Compound (I):the oil phase:the emulsifier:the co-emulsifier:the antioxidant is 0.99-1.98:13-14:55-57:28-29:(greater than 0 to 0.05), e.g., 0.99:13-14:55-57:28-29:(0.01-0.03), preferably 0.99:13.86:56.42:28.71:0.02; wherein the oil phase is medium-chain triglycerides; the emulsifier is polyoxyl-40 hydrogenated castor oil; the co-emulsifier is polyethylene glycol 400; the antioxidant is butylated hydroxytoluene; or
   the self-emulsifying pharmaceutical composition is consisted of Compound (I), the oil phase, the emulsifier, the co-emulsifier and the antioxidant, wherein the weight ratio of Compound (I):the oil phase:the emulsifier:the co-emulsifier:the antioxidant is 0.99-1.98:10-12:59-61:26-28:(0.1-0.5), e.g., 0.99:10-12:59-61:26-28:(0.4-0.5), preferably 0.99:10.90:60.39:27.22:0.50; wherein the oil phase is medium-chain triglycerides; the emulsifier is polyoxyl-40 hydrogenated castor oil; the co-emulsifier is diethylene glycol monoethyl ether; the antioxidant is vitamin E; or
   the self-emulsifying pharmaceutical composition is consisted of Compound (I), the emulsifier and the co-emulsifier, wherein the weight ratio of Compound (I):the emulsifier:the co-emulsifier is 0.99:60-80:20-30, e.g., 0.99:70-80:20-30, or 0.99:75-80:25-30, or 0.99:70-75:20-25, or 0.99:74-76:24-26, or 0.99:74.26:24.75; wherein the emulsifier is polyoxyl-40 hydrogenated castor oil; the co-emulsifier is polyethylene glycol 400 or diethylene glycol monoethyl ether.
Solution 10. The self-emulsifying pharmaceutical composition of Compound (I) according to any one of the preceding solutions, characterised in that the self-emulsifying pharmaceutical composition is diluted to obtain a self-emulsified microemulsion, which has an average particle size of greater than 0 to 250nm, preferably greater than 0 to 100nm, or 10-100 nm, more preferably greater than 0 to 50nm, further preferably 10-50nm, still further preferably 10-40nm, or 20-40nm; preferably, the diluent used for dilution can be selected from water, hydrochloric acid medium (pH = 1.0), acetate buffer (pH=4.5), and phosphate buffer (pH=6.8); more preferably, the dilution ratio is a ratio of the self-emulsifying pharmaceutical composition to the diluent of 1:(3-500), e.g., 1:(5-500).
Solution 11. A self-emulsifying preparation of compound (I), which is prepared from the self-emulsifying pharmaceutical composition of Compound (I) according to any one of the preceding solutions, characterised in that the dosage form of the self-emulsifying preparation of Compound (I) is an oral liquid, a capsule or a tablet, and the capsule can be a soft capsule or a hard capsule; preferably, the dosage form of the self-emulsifying preparation of Compound (I) is an oral liquid.
Solution 12. A process for preparing the self-emulsifying preparation of Compound (I) according to Solution 11, which comprises: adding excipients of the self-emulsifying pharmaceutical composition into a liquid preparation tank, heating and stirring evenly, adding Compound (I) and continuing heating and stirring until it is completely dissolved to obtain a self-emulsifying pharmaceutical composition of Compound (I), wherein the excipients of the self-emulsifying pharmaceutical composition include an oil phase, an emulsifier, a co-emulsifier, and optionally, an antioxidant; preferably the temperature for heating and stirring is 40-70°C;
the obtained self-emulsifying pharmaceutical composition is packaged into oral liquid bottles or filled into capsules, or the self-emulsifying pharmaceutical composition is prepared into granules or pellets by liquid solidification technology and compressed into tablets or capsules, to obtain a self-emulsifying preparation of Compound (I).
Solution 13. Use of the self-emulsifying preparation of Compound (I) according to solution 11 in manufacture of a drug for preventing and/or treating abnormal vascular proliferation-related diseases, and/or a drug as VEGFR-2 inhibitor, and/or a drug for treating CSF1R kinase signal transduction pathway-related diseases, and/or a drug as CSF1R kinase inhibitor;
   preferably, the abnormal vascular proliferation-related disease is selected from tumour, rheumatoid arthritis, age-related macular degeneration, and psoriasis;
   more preferably, said tumour is preferably selected from lung cancer, breast cancer, colorectal cancer, prostate cancer, pancreatic cancer, stomach cancer, liver cancer, ovarian cancer, renal cancer, glioma, melanoma, pancreatic cancer, head and neck cancer, bladder cancer, cervix cancer, cholangiocarcinoma, nasopharyngeal carcinoma, thyroid carcinoma, osteosarcoma, synovial sarcoma, rhabdomyosarcoma, fibrosarcoma, leiomyosarcoma, myeloma, brain glioma, brain metastasis, meningioma, and lymphadenoma;
   particularly preferably, said glioma is glioblastoma, astroglioma, or medulloblastoma.
Solution 14. The self-emulsifying preparation of Compound (I) according to solution 11 for use in preventing and/or treating abnormal vascular proliferation-related diseases, and/or treating CSF1R kinase signal transduction pathway-related diseases;
   preferably, the abnormal vascular proliferation-related disease is selected from tumour, rheumatoid arthritis, age-related macular degeneration, and psoriasis;
   more preferably, said tumour is preferably selected from lung cancer, breast cancer, colorectal cancer, prostate cancer, pancreatic cancer, stomach cancer, liver cancer, ovarian cancer, renal cancer, glioma, melanoma, pancreatic cancer, head and neck cancer, bladder cancer, cervix cancer, cholangiocarcinoma, nasopharyngeal carcinoma, thyroid carcinoma, osteosarcoma, synovial sarcoma, rhabdomyosarcoma, fibrosarcoma, leiomyosarcoma, myeloma, brain glioma, brain metastasis, meningioma, and lymphadenoma;
   particularly preferably, said glioma is glioblastoma, astroglioma, or medulloblastoma.
Solution 15. A method for preventing and/or treating abnormal vascular proliferation-related diseases, and/or treating CSF1R kinase signal transduction pathway-related diseases, which comprises administering to a subject a therapeutically effective amount of the self-emulsifying preparation of Compound (I) according to solution 11;
   preferably, the abnormal vascular proliferation-related disease is selected from tumour, rheumatoid arthritis, age-related macular degeneration, and psoriasis;
   more preferably, said tumour is preferably selected from lung cancer, breast cancer, colorectal cancer, prostate cancer, pancreatic cancer, stomach cancer, liver cancer, ovarian cancer, renal cancer, glioma, melanoma, pancreatic cancer, head and neck cancer, bladder cancer, cervix cancer, cholangiocarcinoma, nasopharyngeal carcinoma, thyroid carcinoma, osteosarcoma, synovial sarcoma, rhabdomyosarcoma, fibrosarcoma, leiomyosarcoma, myeloma, brain glioma, brain metastasis, meningioma, and lymphadenoma;
   particularly preferably, said glioma is glioblastoma, astroglioma, or medulloblastoma.

The present invention has the following beneficial effects by preparing compound (I) into a self-emulsifying preparation:
(1) After oral administration, microemulsions can quickly form, which improves the solubility and dissolution rate of the drug and the penetration rate of the drug through the mucous membrane of the gastrointestinal tract, thereby greatly improving the oral availability of the drug.
(2) The self-emulsifying preparation of compound (I) of the present invention is stable in properties and convenient for long-term storage.
(3) The self-emulsifying pharmaceutical composition of Compound (I) of the present invention can be prepared into various dosage forms such as oral liquids, capsules, and tablets. The dosage forms are easy to use and convenient for patients to take, and have good patient compliance.
(4) The process for preparing the self-emulsifying preparation of Compound (I) of the present invention is simple and convenient for industrial-scale production.

### Detailed description

The present invention is further described below in connection with specific examples. It should be understood that these examples are intended to illustrate the present invention only and are not intended to limit the scope of the present invention. Experimental methods for which no specific conditions are indicated in the following examples are generally in accordance with conventional conditions or in accordance with the conditions recommended by the manufacturers.

The materials used in the examples include the following pharmaceutical grade excipients, which comply with the provisions of China Pharmacopoeia 2020 and/or EUROPEAN PHARMACOPOEIA 11.0:

| | |
|---|---|
| Polyoxyl-15 hydroxystearate | Macrogol 15 hydroxystearate |
| | Source: BASF |
| | Specification: Pharmaceutical grade |
| PH101 | Microcrystalline Cellulose PH101 |
| | Source: Asahi Kasei, Japan |
| | Specification: Pharmaceutical grade (Average particle size: 50µm, bulk density: 0.26-0.31g/cm³) |
| PH302 | Microcrystalline Cellulose PH-302 |
| | Source: Asahi Kasei, Japan |
| | Specification: Pharmaceutical grade (Average particle size: 90µm, bulk density: 0.35-0.46g/cm³) |
| SDS | Sodium dodecyl sulfate |
| | Source: Hunan ER-KANG Pharmaceutical Co Ltd |
| | Specification: Pharmaceutical grade |
| Tween 80 | Polysorbate 80 |
| | Source: Nanjing Well Pharmaceutical Group Co Ltd |
| | Specification: Pharmaceutical grade |
| Butylated hydroxytoluene | Source: Jiangxi A'er Fagaoke Pharmaceutical Industry Limited Company |
| | Specification: Pharmaceutical grade |
| Diethylene glycol monoethyl ether | Source: Gattefossé |
| | Purity: >99.9% |
| | Specification: Pharmaceutical grade |
| Mannitol | Source: Roquette France |
| | PEARLITOL^{®} 200 SD |
| | Content: 98.0-102.0% |
| | Specification: Pharmaceutical grade |
| Croscarmellose sodium | Source: DuPont |
| | Specification: Pharmaceutical grade (not greater than 2% cannot pass through a 200-mesh sieve, and not greater than 10% cannot pass through a 325-mesh sieve, bulk density: 0.529g/cm³) |
| Polyoxyl-35 castor oil | Source: BASF |
| | Specification: Pharmaceutical grade |
| Polyoxyl-40 hydrogenated castor oil | Source: BASF |
| | Specification: Pharmaceutical grade |
| Polyethylene glycol 1500 | Source: Liaoning Oxiranchem Inc. |
| | Specification: Pharmaceutical grade |
| Polyethylene glycol 400 | PEG 400 |
| | Source: Nanjing Well Pharmaceutical Group Co Ltd |
| | Specification: Pharmaceutical grade |
| Normal saline | Home-made, 0.90 wt% sodium chloride (for injection) + the balance of sterile water for injection |
| Vitamin E | Source: Zhejiang Medicine Co., Ltd. |
| | Specification: Pharmaceutical grade |
| Tocopherol polyethylene glycol succinate | Source: Jiangxi Lianlu Biotechnology Co., Ltd. |
| | Specification: Pharmaceutical grade |
| Ethanol | Source: Nanjing Chemical Reagent Co., Ltd. |
| | Specification: Pharmaceutical grade |
| Ethyl acetate | Source: Tianjin Kermel Chemical Reagent Co., Ltd. |
| | Specification: Reagent grade |
| Magnesium stearate | Source: Liaoning Aoda |
| | Specification: Pharmaceutical grade |
| Oleic acid | Source: Nanjing Well Pharmaceutical Group Co Ltd |
| | Specification: Pharmaceutical grade |
| Medium-chain triglycerides (MCT) | Medium-Chain Triglycerides |
| | Source: Liaoning Shinsun Pharmaceutical Co., Ltd. |
| | Specification: Pharmaceutical grade |

### Examples 1-5: Preparation of oral liquids

Specification: 20mL (density about 1.06 g/mL, the same below): 200mg/bottle, 1000 bottles/batch

| Components | Weight percent (%) | | | | |
|---|---|---|---|---|---|
| | Example 1 | Example 2 | Example 3 | Example 4 | Example 5 |
| Compound (I) | 0.99 | 0.99 | 0.99 | 0.99 | 0.99 |
| Medium-chain triglycerides | 13.86 | 13.86 | 13.86 | NA | NA |
| Polyoxyl-40 hydrogenated castor oil | 56.42 | 56.44 | 56.44 | 74.26 | 74.26 |
| Polyethylene glycol 400 | 28.71 | 28.71 | NA | 24.75 | NA |
| Diethylene glycol monoethyl ether | NA | NA | 28.71 | NA | 24.75 |
| Butylated hydroxytoluene | 0.020 | NA | NA | NA | NA |

Preparation process:
According to the above formulae, polyoxyl-40 hydrogenated castor oil melted in warm water at about 60°C to a liquid state in advance. Medium-chain triglycerides, polyoxyl-40 hydrogenated castor oil, polyethylene glycol 400 or diethylene glycol monoethyl ether, and butylated hydroxytoluene were added to a liquid preparation tank and stirred evenly at 40-70°C. Compound (I) was added, and heating and stirring at 40-70°C were continued until the raw materials were completely dissolved to obtain a self-emulsifying liquid, which was then dispensed into oral liquid bottles according to the specification.

The self-emulsifying liquid was taken and diluted according to the proportions of self-emulsifying liquid:water (volume ratio)=1:3, 1:5, 1:20, and 1:500, shaken for 1-2 minutes to obtain the microemulsions. The particle sizes of the microemulsions were measured, and the average particle sizes were in the range of 20-40 nm.

The self-emulsifying liquid was taken and diluted according to the proportions of self-emulsifying liquid:medium (volume ratio)=1:5, 1:20, and 1:500, shaken for 1-2 minutes to obtain the microemulsions. The particle sizes of the microemulsions were measured, and the average particle sizes were in the range of 20-40 nm. The medium could be hydrochloric acid medium (pH=1.0), acetate buffer (pH=4.5) or phosphate buffer (pH=6.8).

The medium was prepared according to Technical Guidelines for Dissolution Testing of Common Oral Solid Preparations (China Food and Drug Administration, 2015).

### Examples 6-7: Preparation of capsules

Specification: 15mg, 10,000 capsules/batch

| Components | Weight percent (%) | |
|---|---|---|
| | Example 6 | Example 7 |
| Compound (I) | 0.99 | 1.98 |
| Medium-chain triglycerides | 13.86 | 13.72 |
| Polyoxyl-40 hydrogenated castor oil | 56.44 | 55.88 |
| Polyethylene glycol 400 | 28.71 | 28.42 |

Preparation process:
According to the above formulae, polyoxyl-40 hydrogenated castor oil was melted in warm water at about 60°C to a liquid state in advance. Medium-chain triglycerides, polyoxyl-40 hydrogenated castor oil, and polyethylene glycol 400 were added to a liquid preparation tank, stirred evenly at 40-70°C.

Compound (I) was added, and the mixture was heated and stirred until the raw materials were completely dissolved to obtain a self-emulsifying liquid. The self-emulsifying liquid was filled into hypromellose capsules, followed by sealing the capsules by dissolving hypromellose with ethanol/water.

### Examples 8-12: Preparation of other oral liquids

### Specification: 20mL: 200mg/bottle, 10 bottles/batch

The following oral liquids were prepared according to the preparation processes of Examples 1-5.

| Components | Weight percent (%) | | | | |
|---|---|---|---|---|---|
| | Example 8 | Example 9 | Example 10 | Example 11 | Example 12 |
| Compound (I) | 0.99 | 0.99 | 0.99 | 0.99 | 0.99 |
| Medium-chain triglycerides | 10.90 | 13.86 | 13.86 | 13.86 | 13.86 |
| Polyoxyl-40 hydrogenated castor oil | 60.39 | NA | NA | NA | NA |
| Tocopherol polyethylene glycol succinate | NA | 56.44 | 56.44 | NA | NA |
| Polyoxyl-15 hydroxystearate | NA | NA | NA | 56.44 | NA |
| Polyoxyl-35 castor oil | NA | NA | NA | NA | 56.44 |
| Polyethylene glycol 400 | NA | 28.71 | NA | 28.71 | 28.71 |
| Diethylene glycol monoethyl ether | 27.22 | NA | 28.71 | NA | NA |
| Vitamin E | 0.50 | NA | NA | NA | NA |

The self-emulsifying liquid was taken and diluted according to the proportions of self-emulsifying liquid:water (volume ratio)=1:3, 1:5, 1:20, and 1:500, shaken for 1-2 minutes to obtain the microemulsions. The particle sizes of the microemulsions were measured, and the average particle sizes were in the range of 10-40 nm.

The self-emulsifying liquid was taken and diluted according to the proportions of self-emulsifying liquid:medium (volume ratio)=1:5, 1:20, and 1:500, shaken for 1-2 minutes to obtain the microemulsions. The particle sizes of the microemulsions were measured, and the average particle sizes were in the range of 10-40 nm. The medium could be hydrochloric acid medium (pH=1.0), acetate buffer (pH=4.5) or phosphate buffer (pH=6.8).

The medium was prepared according to Technical Guidelines for Dissolution Testing of Common Oral Solid Preparations (China Food and Drug Administration, 2015).

### Example 13: Preparation of oral liquid

According to the preparation processes of oral liquids according to Examples 1-5, an oral liquid was prepared according to the following formula and specification.

Specification: 2.0g: 25mg/bottle, 1000 bottles/batch

| Component | Weight percent (%) |
|---|---|
| Compound (I) | 1.25 |
| Medium-chain triglycerides | 13.83 |
| Polyoxyl-40 hydrogenated castor oil | 56.25 |
| Polyethylene glycol 400 | 28.67 |

The above oral liquid can also be packaged into other specifications as needed, e.g., 1.2g:15mg/bottle, or 1.6g:20mg/bottle.

The self-emulsifying liquid was taken and diluted according to the proportions of self-emulsifying liquid:water (volume ratio)=1:3, 1:5, 1:20, and 1:500, shaken for 1-2 minutes to obtain the microemulsions. The particle sizes of the microemulsions were measured, and the average particle sizes were in the range of 20-40 nm.

The self-emulsifying liquid was taken and diluted according to the proportions of self-emulsifying liquid:medium (volume ratio)=1:5, 1:20, and 1:500, shaken for 1-2 minutes to obtain the microemulsions. The particle sizes of the microemulsions were measured, and the average particle sizes were in the range of 20-40 nm. The medium could be hydrochloric acid medium (pH=1.0), acetate buffer (pH=4.5) or phosphate buffer (pH=6.8).

The medium was prepared according to Technical Guidelines for Dissolution Testing of Common Oral Solid Preparations (China Food and Drug Administration, 2015).

Comparative Example 1: Solubilization of API

| Component | Weight percent (%) |
|---|---|
| Compound (I) | 0.99 |
| Ethanol: Tween 80:PEG 400:normal saline (2:5:20:73) | 99.01 |

Preparation process:
Compound (I) was dissolved in ethanol:Tween 80:PEG 400:normal saline (2:5:20:73).

Comparative Example 2: common oral solid preparation

| Component | Weight percent (%) |
|---|---|
| Compound (I) | 15 |
| Mannitol 200SD | 74.5 |
| PH302 | 10 |
| SDS | 0.5 |

Preparation process:
The components were weighed according to the formula. Compound (I) was jet-milled and sieved through a 60-mesh sieve. The components were uniformly mixed using the equal amount method to prepare drug-containing granules with a drug content of 15mg/100mg.

Comparative Example 3:

| Component | Amount | Weight percent (%) |
|---|---|---|
| Compound (I) | 60mg | 1.02 |
| Medium-chain triglycerides | 1 mL | 15.63 |
| Polyoxyl-40 hydrogenated castor oil | 1.5 mL | 26.10 |
| Diethylene glycol monoethyl ether | 2.5 mL | 42.01 |
| Oleic acid | 1 mL | 15.24 |
| Ethanol:ethyl acetate (1:1) | 15-20 mL | N/A |

Preparation process:
Compound (I) was dissolved in a mixed solvent of ethanol and ethyl acetate (1:1), heated and ultrasonicated until fully dissolved. The resulting solution was filtered through a 0.45 µm organic filter membrane to obtain the drug solution, which was reserved for subsequent use. Polyoxyl-40 hydrogenated castor oil melted in warm water at about 60°C in advance to achieve a liquid state. The oil phase was then uniformly mixed with other excipients according to the aforementioned ratio. The drug solution was added to the mixture, followed by stirring at room temperature for 30 minutes to ensure homogeneity. The organic solvent was removed by rotary evaporation under reduced pressure in a 65°C water bath to produce a drug-loaded light-yellow oily liquid. The resulting liquid was filled into oral liquid bottles.

Assay 1: stability test
(1) The oral liquids obtained in Examples 1-5, 8-9 and 13 and Comparative Example 3, and the liquid capsule preparations obtained in Examples 6-7 were stored at high temperatures of 40°C and 60°C for 10 days. The test results were as follows:

**Table 1: Comparison of the preparations of Examples 1-9 and 13 and Comparative Example 3 during the storage procedure for stability**

| Group | Content requirements in the storage procedure for stability | Day 0 | Test at high temperature 40°C | | | Test at high temperature 60°C | |
|---|---|---|---|---|---|---|---|
| | | | Day 5 | Day 10 | Day 30 | Day 5 | Day 10 |
| Example 1 | Significant change in the preparation quality includes: the content differs from the initial value by 5% or above | 102.6% | 102.1% | 102.1% | 101.3% | 101.1% | 103.3% |
| Example 2 | | 101.6% | 100.8% | 102.3% | 101.5% | 101.2% | 100.3% |
| Example 3 | | 99.8% | 100.5% | 99.8% | 98.9% | 98.7% | 96.9% |
| Example 4 | | 100.8% | 99.8% | 97.6% | 98.8% | 99.6% | 98.2% |
| Example 5 | | 100.5% | 98.2% | 99.1% | 97.8% | 98.6% | 97.8% |
| Example 6 | | 100.9% | 100.3% | 99.6% | 98.1% | 99.7% | 99.2% |
| Example 7 | | 99.6% | 100.2% | 98.2% | 97.9% | 99.0% | 98.2% |
| Example 8 | | 100.3% | 98.6% | 97.2% | 96.2% | 97.5% | 96.0% |
| Example 9 | | 100.5% | 99.8% | 98.5% | 97.2% | 99.6% | 98.3% |
| Example 13 | | 101.5% | N/A | N/A | 100.9% | 100.9% | 99.2% |
| Comparative Example 3 | | 100.0% | 98.6% | 93.1% | N/A | 90.7% | 89.3% |

| | | | | | | | |
|---|---|---|---|---|---|---|---|
| N/A: Not Available | | | | | | | |

The test results demonstrated that during the storage procedure for stability, no significant changes in content and appearance were observed for the preparations of Examples 1-9 and 13 after being stored at high temperatures of 40°C and 60°C for 10 days, indicating stable product quality. The content of the preparation of Comparative Example 3 decreased by 5% or higher after being stored at high temperatures of 40°C and 60°C for 10 days, and the preparation underwent a significant quality change. In addition, the preparation of Comparative Example 3 changed from a clear and transparent yellow liquid to a pink liquid during the storage procedure.
(2) The preparation of Example 1 was stored at a temperature of 2-8°C to survey the stability. The results were as follows:

**Table 2: Changes in appearance and content of the preparation of Example 1 during storage at 2-8°C**

| Item | Limit requirement | Time (month) | | | | | | | |
|---|---|---|---|---|---|---|---|---|---|
| | | 0 | 3 | 6 | 9 | 12 | 18 | 24 | 36 |
| Appearance | A white to yellow oily liquid; after heating in a water bath, it turns into a clear and transparent yellow solution | Meeting the requirement | | | | | | | |
| Content (%) | 90.0%-110.0%, and during the storage procedure for stability, the content differs from the initial value by less than 5% | 103.2 | 101.4 | 102.0 | 103.4 | 102.1 | 102.7 | 101.3 | 103.5 |

The test results demonstrated that no significant changes in content and appearance were observed for the preparation of Example 1 after being stored at a temperature of 2-8°C for 24 months or even for 36 months, indicating stable product quality.
(3) The preparation of Example 13 was stored respectively at 2-8°C, 30°C±2°C/65%RH±5%RH, and 40°C±2°C/75%RH±5%RH to survey the stability. The results were as follows:

**Table 3: Changes in appearance and content of the preparation of Example 13 during storage at 2-8°C**

| Item | Limit requirement | Time (month) | | |
|---|---|---|---|---|
| | | 0 | 3 | 6 |
| Appearance | A yellow-green to yellow paste-like semisolid or liquid; after diluting with water, it becomes a clear liquid with slight opalescence | Meeting the requirement | Meeting the requirement | Meeting the requirement |
| Content | 90.0%-110.0%, and during the storage procedure for stability, the content differs from the initial value by less than 5% | 102.3% | 100.0% | 99.5% |

**Table 4: Changes in appearance and content of the preparation of Example 13 during storage at 30°C±2°C/65%RH±5%RH**

| Item | Limit requirement | Time (month) | | |
|---|---|---|---|---|
| | | 0 | 3 | 6 |
| Appearance | A yellow-green to yellow paste-like semisolid or liquid; after diluting with water, it becomes a clear liquid with slight opalescence | Meeting the requirement | Meeting the requirement | Meeting the requirement |
| Content | 90.0%-110.0%, and during the storage procedure for stability, the content differs from the initial value by less than 5% | 102.3% | 101.9% | 100.2% |

**Table 5: Changes in appearance and content of the preparation of Example 13 during storage at 40°C±2°C/75%RH±5%RH**

| Item | Limit requirement | Time (month) | | |
|---|---|---|---|---|
| | | 0 | 3 | 6 |
| Appearance | A yellow-green to yellow paste-like semisolid or liquid; after diluting with water, it becomes a clear liquid with slight opalescence | Meeting the requirement | Meeting the requirement | Meeting the requirement |
| Content | 90.0%-110.0%, and during the storage procedure for stability, the content differs from the initial value by less than 5% | 102.3% | 101.4% | 99.8% |

The test results demonstrated that no significant changes in content and appearance were observed for the preparation of Example 13 after being stored at conditions of 2-8°C, 30°C±2°C/65%RH±5%RH and 40°C±2°C/75%RH±5%RH, indicating stable product quality.

### Assay 2: Pharmacokinetic test of the preparations of Examples 1-5 and 13

In the following assays, when the tested preparation was an oral liquid, the oral liquid was taken to a specified volume, then diluted with about 3 to 5 times the volume of water, shaken or stirred until the liquid became clear, and then administered to the subject; when the tested preparation was a capsule, the capsule was administered to the subject with the same amount of water as used for dilution.

### (1) Pharmacokinetic test of the preparations of Examples 1-5

Twenty male Sprague Dawley rats (SD rats) (5 groups, 4 rats per group) were administered the tested preparation at a dose of 15 mg/kg by gavage. Blood samples (0.2 mL) were collected via orbital puncture from each experimental animal at 0h, 0.25h, 0.5h, 1h, 2h, 4h, 6h, 8h, and 24h after administration. Plasma was obtained by centrifugation at 4°C and 3000×g. The concentration of Compound (I) in rat plasma was determined by LC-MS/MS. The pharmacokinetic parameters were calculated using the non-compartmental model of Phoenix WinNonlin 8.0 based on the plasma drug concentration data. The results are as follows:

**Table 6: Results of the rat pharmacokinetic test of the preparations of Examples 1-5**

| Group | Pharmacokinetic parameters (n=4) | | | |
|---|---|---|---|---|
| | Tₘₐₓ | Cₘₐₓ | AUC₀₋ₜ | AUC_{0-∞} |
| | h | ng/mL | h*ng/mL | h*ng/mL |
| Example 1 | 2.00 | 1665 | 11776 | 11828 |
| Example 2 | 2.00 | 1758 | 12853 | 12898 |
| Example 3 | 1.75 | 1463 | 11590 | 11630 |
| Example 4 | 2.50 | 1758 | 12427 | 12480 |
| Example 5 | 2.00 | 1800 | 11875 | 11931 |

| | | | | |
|---|---|---|---|---|
| In the above table, t in AUC₀₋ₜ refers to the endpoint time (24 h). | | | | |

### (2) Pharmacokinetic test of the preparation of Example 13

According to the pharmacokinetic test of the preparations of Examples 1-5, a pharmacokinetic test was conducted on the preparation of Example 13 using male Sprague Dawley rats (4 SD rats). The results are as follows:

**Table 7: Results of the rat pharmacokinetic test of the preparation of Example 13**

| Group | Pharmacokinetic parameters (n=4) | | | |
|---|---|---|---|---|
| | Tₘₐₓ | Cₘₐₓ | AUC₀₋ₜ | AUC_{0-∞} |
| | h | ng/mL | h*ng/mL | h*ng/mL |
| Example 13 | 2.00 | 2485 | 13832 | 14123 |

| | | | | |
|---|---|---|---|---|
| In the above table, t in AUC₀₋ₜ refers to the endpoint time (24 h). | | | | |

### Assay 3: Pharmacokinetic test of the preparations of Examples 2 and 6-7

Beagle dogs were orally administered the tested preparation at a dose of 15 mg/dog (18 dogs, 6 dogs per group). Blood samples (2 mL) were collected from the forelimb vein of each experimental animal at 0h, 0.15h, 0.5h, 1h, 2h, 4h, 6h, 8h, 10h, and 24h after administration. Plasma was obtained by centrifugation at 4°C and 3000×g. The concentration of Compound (I) in dog plasma was determined by LC-MS/MS. The pharmacokinetic parameters were calculated using the non-compartmental model of Phoenix WinNonlin 8.0 based on the plasma drug concentration data. The results are as follows:

**Table 8: Results of the Beagle dog pharmacokinetic test of the preparations of Examples 2, 6 and 7**

| Group | Pharmacokinetic parameters (n=6) | | | |
|---|---|---|---|---|
| | Tₘₐₓ | Cₘₐₓ | AUC₀₋ₜ | AUC_{0-∞} |
| | h | ng/mL | h*ng/mL | h*ng/mL |
| Example 2 | 1 (0.5, 2) | 124±25 (20.2) | 413±96 (23.1) | 424±95 (22.4) |
| Example 6 | 1 (1, 2) | 186±75 (40.6) | 611±353 (57.7) | 616±354 (57.5) |
| Example 7 | 1 (1, 2) | 165±96 (58.3) | 546±387 (70.9) | 552±388 (70.3) |
| Note: Tₘₐₓ: median (maximum value, minimum value); Cₘₐₓ, AUC₀₋ₜ, AUC_{0-∞}: Average±SD(RSD) | | | | |

| | | | | |
|---|---|---|---|---|
| In the above table, t in AUC₀₋ₜ refers to the endpoint time (24 h). | | | | |

### Assay 4: Pharmacokinetic test of Comparative Example

Nine male Sprague Dawley rats (SD rats) (3 groups, 3 rats per group) were administered the tested preparation or API of Comparative Examples 1-3 (wherein Comparative Example 2 was formulated with 0.5% CMC-Na) at a dose of 10 mg/kg by gavage. Blood samples (0.2 mL) were collected via orbital puncture from each experimental animal at 0h, 0.5h, 1h, 2h, 4h, 8h, and 24h after administration. Plasma was obtained by centrifugation at 4°C and 3000×g. The concentration of Compound (I) in rat plasma was determined by LC-MS/MS. The pharmacokinetic parameters were calculated using the non-compartmental model of Phoenix WinNonlin 8.0 based on the plasma drug concentration data. The results are as follows:

**Table 9: Results of the rat pharmacokinetic test of Comparative Examples 1-3 (n=3)**

| Group | Tₘₐₓ (h) | Cₘₐₓ (ng/mL) | AUC₀₋ₜ (h*ng/mL) | AUC_{0-∞} (h*ng/mL) |
|---|---|---|---|---|
| Comparative Example 1 | 2.00 | 357 | 1356 | 1386 |
| Comparative Example 2 | 4.00 | 26.0 | 130 | 144 |
| Comparative Example 3 | 2.67 | 902 | 6299 | 6313 |

| | | | | |
|---|---|---|---|---|
| In the above table, t in AUC₀₋ₜ refers to the endpoint time (24 h). | | | | |

### Assay 5: Human pharmacokinetic test

A clinical study of phase Ia was performed on patients with solid tumours using the preparation of Example 1. During a single administration period, 3 mL of whole blood was collected before administration and 30min±2min, 1h±5min, 2h±5min, 4h±5min, 8h±10min, 12h±10min, 24h±10min, 48h±15min, and 72h±30min after administration, and plasma was obtained after centrifugation. The concentration of the drug in human plasma was determined by the validated LC-MS/MS method, and its pharmacokinetic parameters were calculated using the non-compartmental model of Phoenix WinNonlin 8.3.

The test results showed that after a single oral administration of 15-30 mg, the drugs were rapidly absorbed by the subjects, with the median Tₘₐₓ of the drug in plasma being 2.00-2.01 h, and then the plasma drug concentration was slowly eliminated. The average values of t_{1/2}, apparent distribution volume (Vz/F) and clearance rate (CL/F) of the drug in each dose group were 27.2-30.9 h, 187-189 L and 4.39-5.06 L/h, respectively. In general, the systemic exposure of the drug increased with the increase of the administration dose, with the average Cₘₐₓ values of 156-301 ng/mL, and the average areas under the plasma drug concentration-time curve (AUCₗₐₛₜand AUC_{inf}) of 2680-5910 h*ng/mL and 3300-7420 h*ng/mL, respectively. Within the dose range of 15-30 mg, the drug substantially showed linear pharmacokinetic characteristics *in vivo.*

**Table 10: Summary of main PK parameters after single oral administration to subjects**

| PK parameters (unit) | | Dose Group | |
|---|---|---|---|
| | | 15 mg (N=14) | 30 mg (N=5) |
| Cₘₐₓ (ng/mL) | Mean (%CV) | 156 (20.0) | 301 (18.5) |
| Tₘₐₓ (h) | Median (Min, Max) | 2.01 (0.48, 4.05) | 2.00 (1.97, 2.05) |
| AUCₗₐₛₜ (h*ng/mL) | Mean (%CV) | 2680 (27.7) | 5910 (28.7) |
| AUC_{inf} (h*ng/mL) | Mean (%CV) | 3300 (33.0) | 7420 (37.6) |
| t_{1/2} (h) | Mean (%CV) | 27.2 (22.2) | 30.9 (19.9) |
| CL/F (L/h) | Mean (%CV) | 5.06 (35.7) | 4.39 (26.9) |
| Vz/F (L) | Mean (%CV) | 187 (21.0) | 189 (18.7) |

The above descriptions merely represent the preferred embodiments of the present invention. It should be noted that those skilled in the art may make several improvements and modifications without departing from the principles of the present invention. Such improvements and modifications shall also fall within the protection scope of the present invention.

## Claims

1. A self-emulsifying pharmaceutical composition of Compound (I), comprising: Compound (I), an oil phase, an emulsifier and a co-emulsifier, wherein the weight ratio of Compound (I):the oil phase:the emulsifier:the co-emulsifier is 0.5-2:0-20:50-80:20-35; preferably, 0.99-1.98:0-20:50-80:20-35; more preferably, 0.99:0-20:50-80:20-35, or 1.25:0-20:50-80:20-35, or 1.98:0-20:50-80:20-35;
Compound (I) has a structural formula represented by the following formula (I),

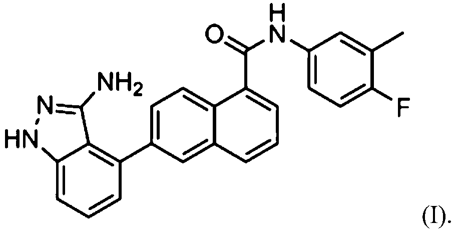

2. The self-emulsifying pharmaceutical composition of Compound (I) according to claim 1, **characterised in that** the weight ratio of Compound (I):the oil phase:the emulsifier:the co-emulsifier is
0.5-2:10-20:50-65:20-30; or
0.5-2:0:60-80:20-30; or
0.5-2:0-15:55-75:20-30;
more preferably
0.99-1.98:0-15:55-75:20-30; or
0.99-1.98:0-15:55-75:24-30; or
0.99-1.98:10-15:55-65:25-30; or
0.99-1.98:13-14:55-57:28-29; or
0.99-1.98:0:70-80:20-30; or
0.99-1.98:0:75-80:25-30; or
0.99-1.98:0:70-75:20-25; or
0.99-1.98:0:74-76:24-26;
further preferably
0.99:10-20:50-65:20-30; or
0.99:10-15:55-65:25-30; or
0.99:13-14:55-57:28-29; or
1.25:10-20:50-65:20-30; or
1.25:10-15:55-65:25-30; or
1.25:13-14:55-57:28-29; or
1.98:10-20:50-65:20-30; or 1.98:10-15:55-65:25-30; or
1.98:13-14:55-57:28-29; or
0.99:0:60-80:20-30; or
0.99:0:70-80:20-30; or
0.99:0:75-80:25-30; or
0.99:0:70-75:20-25; or
0.99:0:74-76:24-26.

3. The self-emulsifying pharmaceutical composition of Compound (I) according to any one of the preceding claims, **characterised in that** the concentration of Compound (I) is 5-20mg/g, preferably 9.9-19.8mg/g, more preferably 9.9mg/g, 19.8mg/g or 12.5mg/g.

4. The self-emulsifying pharmaceutical composition of Compound (I) according to any one of the preceding claims, **characterised in that** the oil phase is selected from medium-chain triglycerides, monocaprylin, dicaprylin, glyceryl monooleate, glyceryl monolinoleate, corn oil, castor oil, sesame oil, peanut oil, and olive oil; preferably medium-chain triglycerides.

5. The self-emulsifying pharmaceutical composition of Compound (I) according to any one of the preceding claims, **characterised in that** the emulsifier is selected from polyoxyl-40 hydrogenated castor oil, polyoxyl-35 castor oil, tocopherol polyethylene glycol succinate, polyoxyl-15 hydroxystearate, linoleoyl polyoxyl-6 glyceride, caprylocaproyl polyoxyl glyceride; preferably polyoxyl-40 hydrogenated castor oil, tocopherol polyethylene glycol succinate, polyoxyl-35 castor oil; more preferably polyoxyl-40 hydrogenated castor oil.

6. The self-emulsifying pharmaceutical composition of Compound (I) according to any one of the preceding claims, **characterised in that** the co-emulsifier is selected from polyethylene glycol, diethylene glycol monoethyl ether, propylene glycol, glycerol; preferably polyethylene glycol 400, diethylene glycol monoethyl ether.

7. The self-emulsifying pharmaceutical composition of Compound (I) according to any one of the preceding claims, **characterised in that** it further optionally contains an antioxidant, preferably, the weight ratio of Compound (I) to the antioxidant is 1:0-0.5, preferably 1:0-0.1, more preferably 1:0-0.05, further preferably 1:0-0.03, still further preferably 1:0.02, 0.99:0.02 or 1:0.

8. The self-emulsifying pharmaceutical composition of Compound (I) according to claim 7, **characterised in that** the antioxidant is selected from butylated hydroxytoluene, butylated hydroxyanisole, vitamin E, propyl gallate; preferably butylated hydroxytoluene, vitamin E, more preferably butylated hydroxytoluene.

9. The self-emulsifying pharmaceutical composition of Compound (I) according to claim 1, **characterised in that** the self-emulsifying pharmaceutical composition is consisted of Compound (I), the oil phase, the emulsifier and the co-emulsifier, wherein the weight ratio of Compound (I):the oil phase:the emulsifier:the co-emulsifier is 0.5-2:10-20:50-65:20-30, preferably, 0.99-1.98:10-15:55-65:25-30, more preferably, 0.99-1.98:13-14:55-57:28-29, e.g., 0.99:13-14:55-57:28-29, or 1.25:13-14:55-57:28-29, or 1.98:13-14:55-57:28-29; wherein the oil phase is medium-chain triglycerides; the emulsifier is polyoxyl-40 hydrogenated castor oil, tocopherol polyethylene glycol succinate, polyoxyl-15 hydroxystearate or polyoxyl-35 castor oil; the co-emulsifier is polyethylene glycol 400 or diethylene glycol monoethyl ether; or
the self-emulsifying pharmaceutical composition is consisted of Compound (I), the oil phase, the emulsifier and the co-emulsifier, wherein the weight ratio of Compound (I):the oil phase:the emulsifier:the co-emulsifier is 0.99-1.98:13-14:55-57:28-29, e.g., 0.99:13-14:55-57:28-29; wherein the oil phase is medium-chain triglycerides; the emulsifier is polyoxyl-40 hydrogenated castor oil; the co-emulsifier is polyethylene glycol 400 or diethylene glycol monoethyl ether; or
the self-emulsifying pharmaceutical composition is consisted of Compound (I), the oil phase, the emulsifier and the co-emulsifier, wherein the weight ratio of Compound (I):the oil phase:the emulsifier:the co-emulsifier is 0.99-1.98:13-14:55-57:28-29, e.g., 0.99:13-14:55-57:28-29; wherein the oil phase is medium-chain triglycerides; the emulsifier is tocopherol polyethylene glycol succinate; the co-emulsifier is polyethylene glycol 400 or diethylene glycol monoethyl ether; or
the self-emulsifying pharmaceutical composition is consisted of Compound (I), the oil phase, the emulsifier and the co-emulsifier, wherein the weight ratio of Compound (I):the oil phase:the emulsifier:the co-emulsifier is 0.99-1.98:13-14:55-57:28-29, e.g., 0.99:13-14:55-57:28-29; wherein the oil phase is medium-chain triglycerides; the emulsifier is polyoxyl-15 hydroxystearate or polyoxyl-35 castor oil; the co-emulsifier is polyethylene glycol 400; or
the self-emulsifying pharmaceutical composition is consisted of Compound (I), the oil phase, the emulsifier and the co-emulsifier, wherein the weight ratio of Compound (I):the oil phase:the emulsifier:the co-emulsifier is 0.99-1.98:13-14:55-57:28-29, e.g., 1.25:13-14:55-57:28-29; wherein the oil phase is medium-chain triglycerides; the emulsifier is polyoxyl-40 hydrogenated castor oil; the co-emulsifier is polyethylene glycol 400; or
the self-emulsifying pharmaceutical composition is consisted of Compound (I), the oil phase, the emulsifier and the co-emulsifier, wherein the weight ratio of Compound (I):the oil phase:the emulsifier:the co-emulsifier is 0.99-1.98:13-14:55-57:28-29, e.g., 0.99:13-14:55-57:28-29, or 1.98:13-14:55-57:28-29; wherein the oil phase is medium-chain triglycerides; the emulsifier is polyoxyl-40 hydrogenated castor oil; the co-emulsifier is polyethylene glycol 400; or
the self-emulsifying pharmaceutical composition is consisted of Compound (I), the oil phase, the emulsifier, the co-emulsifier and the antioxidant, wherein the weight ratio of Compound (I):the oil phase:the emulsifier:the co-emulsifier:the antioxidant is 0.5-2:10-20:50-65:20-30:(greater than 0 to 0.5), preferably, 0.99-1.98:10-15:55-65:25-30:(greater than 0 to 0.5), more preferably, 0.99-1.98:10-15:55-61:27-29:(greater than 0 to 0.5), e.g., 0.99:13-14:55-57:28-29:(0.01-0.03), or 0.99:10-12:59-61:26-28:(0.4-0.5); wherein the oil phase is medium-chain triglycerides; the emulsifier is polyoxyl-40 hydrogenated castor oil, tocopherol polyethylene glycol succinate, polyoxyl-15 hydroxystearate or polyoxyl-35 castor oil; the co-emulsifier is polyethylene glycol 400 or diethylene glycol monoethyl ether; the antioxidant is butylated hydroxytoluene, or vitamin E; or
the self-emulsifying pharmaceutical composition is consisted of Compound (I), the oil phase, the emulsifier, the co-emulsifier and the antioxidant, wherein the weight ratio of Compound (I):the oil phase:the emulsifier:the co-emulsifier:the antioxidant is 0.99-1.98:13-14:55-57:28-29:(greater than 0 to 0.05), e.g., 0.99:13-14:55-57:28-29:(0.01-0.03); wherein the oil phase is medium-chain triglycerides; the emulsifier is polyoxyl-40 hydrogenated castor oil; the co-emulsifier is polyethylene glycol 400; the antioxidant is butylated hydroxytoluene; or
the self-emulsifying pharmaceutical composition is consisted of Compound (I), the oil phase, the emulsifier, the co-emulsifier and the antioxidant, wherein the weight ratio of Compound (I):the oil phase:the emulsifier:the co-emulsifier:the antioxidant is 0.99-1.98:10-12:59-61:26-28:(0.1-0.5), e.g., 0.99:10-12:59-61:26-28:(0.4-0.5); wherein the oil phase is medium-chain triglycerides; the emulsifier is polyoxyl-40 hydrogenated castor oil; the co-emulsifier is diethylene glycol monoethyl ether; the antioxidant is vitamin E; or
the self-emulsifying pharmaceutical composition is consisted of Compound (I), the emulsifier and the co-emulsifier, wherein the weight ratio of Compound (I):the emulsifier:the co-emulsifier is 0.99:60-80:20-30, e.g., 0.99:70-80:20-30, or 0.99:75-80:25-30, or 0.99:70-75:20-25, or 0.99:74-76:24-26; wherein the emulsifier is polyoxyl-40 hydrogenated castor oil; the co-emulsifier is polyethylene glycol 400 or diethylene glycol monoethyl ether.

10. The self-emulsifying pharmaceutical composition of Compound (I) according to any one of the preceding claims, **characterised in that** the self-emulsifying pharmaceutical composition is diluted to obtain a self-emulsified microemulsion, which has an average particle size of greater than 0 to 250nm, preferably 10-100nm.

11. A self-emulsifying preparation of Compound (I), which is prepared from the self-emulsifying pharmaceutical composition of Compound (I) according to any one of the preceding claims, **characterised in that** the dosage form of the self-emulsifying preparation of Compound (I) is an oral liquid, a capsule or a tablet, and the capsule can be a soft capsule or a hard capsule; preferably, the dosage form of the self-emulsifying preparation of Compound (I) is an oral liquid.

12. A process for preparing the self-emulsifying preparation of Compound (I) according to claim 11, which comprises: adding excipients of the self-emulsifying pharmaceutical composition into a liquid preparation tank, heating and stirring evenly, adding Compound (I) and continuing heating and stirring until it is completely dissolved to obtain a self-emulsifying pharmaceutical composition of Compound (I), wherein the excipients of the self-emulsifying pharmaceutical composition include an oil phase, an emulsifier, a co-emulsifier, and optionally, an antioxidant; preferably the temperature for heating and stirring is 40-70°C;
the obtained self-emulsifying pharmaceutical composition is packaged into oral liquid bottles or filled into capsules, or the self-emulsifying pharmaceutical composition is prepared into granules or pellets by liquid solidification technology and compressed into tablets or capsules, to obtain a self-emulsifying preparation of Compound (I).

13. Use of the self-emulsifying preparation of Compound (I) according to claim 11 in manufacture of a drug for preventing and/or treating abnormal vascular proliferation-related diseases, and/or a drug as VEGFR-2 inhibitor, and/or a drug for treating CSF1R kinase signal transduction pathway-related diseases, and/or a drug as CSF1R kinase inhibitor;
preferably, the abnormal vascular proliferation-related disease is selected from tumour, rheumatoid arthritis, age-related macular degeneration, and psoriasis;
more preferably, said tumour is preferably selected from lung cancer, breast cancer, colorectal cancer, prostate cancer, pancreatic cancer, stomach cancer, liver cancer, ovarian cancer, renal cancer, glioma, melanoma, pancreatic cancer, head and neck cancer, bladder cancer, cervix cancer, cholangiocarcinoma, nasopharyngeal carcinoma, thyroid carcinoma, osteosarcoma, synovial sarcoma, rhabdomyosarcoma, fibrosarcoma, leiomyosarcoma, myeloma, brain glioma, brain metastasis, meningioma, and lymphadenoma;
particularly preferably, said glioma is glioblastoma, astroglioma, or medulloblastoma.

14. The self-emulsifying preparation of Compound (I) according to claim 11 for use in preventing and/or treating abnormal vascular proliferation-related diseases, and/or treating CSF1R kinase signal transduction pathway-related diseases;
preferably, the abnormal vascular proliferation-related disease is selected from tumour, rheumatoid arthritis, age-related macular degeneration, and psoriasis;
more preferably, said tumour is preferably selected from lung cancer, breast cancer, colorectal cancer, prostate cancer, pancreatic cancer, stomach cancer, liver cancer, ovarian cancer, renal cancer, glioma, melanoma, pancreatic cancer, head and neck cancer, bladder cancer, cervix cancer, cholangiocarcinoma, nasopharyngeal carcinoma, thyroid carcinoma, osteosarcoma, synovial sarcoma, rhabdomyosarcoma, fibrosarcoma, leiomyosarcoma, myeloma, brain glioma, brain metastasis, meningioma, and lymphadenoma;
particularly preferably, said glioma is glioblastoma, astroglioma, or medulloblastoma.

15. A method for preventing and/or treating abnormal vascular proliferation-related diseases, and/or treating CSF1R kinase signal transduction pathway-related diseases, which comprises administering to a subject a therapeutically effective amount of the self-emulsifying preparation of Compound (I) according to claim 11;
preferably, the abnormal vascular proliferation-related disease is selected from tumour, rheumatoid arthritis, age-related macular degeneration, and psoriasis;
more preferably, said tumour is preferably selected from lung cancer, breast cancer, colorectal cancer, prostate cancer, pancreatic cancer, stomach cancer, liver cancer, ovarian cancer, renal cancer, glioma, melanoma, pancreatic cancer, head and neck cancer, bladder cancer, cervix cancer, cholangiocarcinoma, nasopharyngeal carcinoma, thyroid carcinoma, osteosarcoma, synovial sarcoma, rhabdomyosarcoma, fibrosarcoma, leiomyosarcoma, myeloma, brain glioma, brain metastasis, meningioma, and lymphadenoma;
particularly preferably, said glioma is glioblastoma, astroglioma, or medulloblastoma.
